(19) Europäisches Patentamt
European Patent Office
Office européen des brevets

(11) Veröffentlichungsnummer: **0 062 612**
**A1**

# (12) EUROPÄISCHE PATENTANMELDUNG

(21) Anmeldenummer: **82810141.0**

(22) Anmeldetag: **29.03.82**

(51) Int. Cl.³: **C 07 D 417/04**, A 01 N 43/78

(30) Priorität: **02.04.81 CH 2252/81**

(43) Veröffentlichungstag der Anmeldung: **13.10.82**
**Patentblatt 82/41**

(84) Benannte Vertragsstaaten: **AT BE CH DE FR IT LI NL**

(71) Anmelder: **CIBA-GEIGY AG, Patentabteilung Postfach, CH-4002 Basel (CH)**

(72) Erfinder: **Beriger, Ernst, Dr., Grabenmattweg 29, CH-4123 Allschwil (CH)**

(54) **Neue Thiazolinderivate.**

(57) 2-[Pyridyl-(3')]-thiazolin der Formel

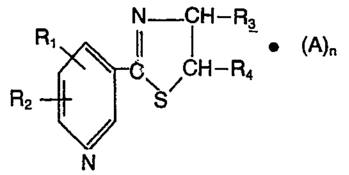

(I),

worin $R_1$ und $R_2$ je Wasserstoff, Halogen oder $C_1$-$C_4$-Alkyl, $R_3$ und $R_4$ je Wasserstoff oder $C_1$-$C_4$-Alkyl, A eine anorganische oder organische Säure und n die Zahlen 0, 1 oder 2 bedeuten, mit der Massgabe, dass $R_1$, $R_2$, $R_3$ und $R_4$ nicht zusammen Wasserstoff sein dürfen.

Es werden Verfahren zur Herstellung dieser 2-[Pyridyl-(3')]-thiazoline und ihre Verwendung sowie die Verwendung des 2-[Pyridyl-(3')]-thiazolins und dessen Salze mit anorganischen oder organischen Säuren beschrieben.

EP 0 062 612 A1

CIBA-GEIGY AG
Basel (Schweiz)

5-13362/+

## Neue Thiazolinderivate

Die vorliegende Erfindung betrifft 2-[Pyridyl-(3')]-thiazoline und ihre tertiären und quartären Salze, Verfahren zu ihrer Herstellung und ihre Verwendung in der Schädlingsbekämpfung.

Die 2-[Pyridyl-(3')]-thiazoline haben die Formel

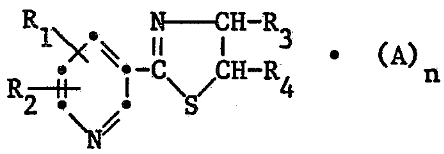

(I) ,

worin $R_1$ und $R_2$ je Wasserstoff, Halogen oder $C_1$-$C_4$-Alkyl,
$R_3$ und $R_4$ je Wasserstoff oder $C_1$-$C_4$-Alkyl,
A eine anorganische oder organische Säure und
n die Zahlen 0, 1 oder 2 bedeuten, mit der Massgabe, dass
$R_1$, $R_2$, $R_3$ und $R_4$ nicht zusammen Wasserstoff sein dürfen.

Unter Halogen ist dabei Fluor, Chlor, Brom oder Jod, insbesondere aber Chlor, zu verstehen. Die $C_1$-$C_4$-Alkylgruppen bei $R_1$ bis $R_4$ sind Methyl, Aethyl, Propyl, Isopropyl, n-, i-, sek.- oder tert.-Butyl. Bevorzugt bei $R_1$ und $R_2$ ist die Methylgruppe. Für A kommen als anorganische Säuren beispielsweise HCl, $H_2SO_4$, HBr und $H_3PO_4$ und als organische Säuren beispielsweise gesättigte und ungesättigte Mono-, Di- und Tricarbonsäuren wie z.B. Ameisensäure, Essigsäure, Oxalsäure, Phthalsäure, Bernsteinsäure und Zitronensäure in Betracht.

Bevorzugt sind Verbindungen der Formel I, worin $R_1$ Wasserstoff, $R_2$ Chlor oder Methyl und $R_3$ und $R_4$ je Wasserstoff oder $R_1$ und $R_2$ je Wasserstoff, $R_3$ Wasserstoff und $R_4$ Methyl oder Aethyl oder $R_3$ Methyl oder Aethyl, $R_4$ Wasserstoff, A eine anorganische oder organische Säure und n die Zahlen 0, 1 oder 2 bedeuten.

Die Verbindungen der Formel I können nach an sich bekannten Methoden z.B. wie folgt hergestellt werden:

1. 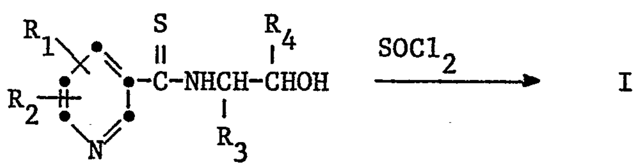


(II)

2. 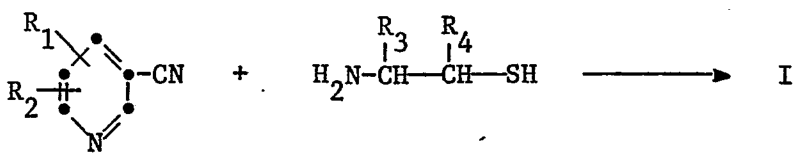

(III) (IV)

In den Formeln II, III und IV haben $R_1$ bis $R_4$ und n die für die Formel I angegebene Bedeutung.

Die Verfahren 1 und 2 werden bei einer Reaktionstemperatur zwischen -50°C und +130°C, vorzugsweise zwischen -10°C und +100°C, bei normalem oder leicht erhöhtem Druck und in Gegenwart eines gegenüber den Reaktionsteilnehmern inerten Lösungs- oder Verdünnungsmittel vorgenommen.

Als Lösungs- und Verdünnungsmittel eignen sich z.B. Aether und ätherartige Verbindungen wie Diäthyläther, Di-isopropyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die Ausgangssfoffe der Formeln II, III und IV sind bekannt bzw. können analog bekannten Methoden hergestellt werden.

Die Verbindungen der Formel I sowie das 2-[Pyridyl-(3')]-thiazolin und dessen Salze eignen sich zur Bekämpfung von Schädlingen an Tieren und Pflanzen.

So eignen sich die Verbindungen der Formel I sowie das 2-[Pyridyl-(3')]-thiazolin und dessen Salze zur Bekämpfung von Insekten z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopathogenen Milben und Zecken der Ordnung Akarina.

In diesem Zusammenhang ist hervorzuheben, dass die genannten Verbindungen sich sowohl durch eine stark ausgeprägte systemische als auch Kontakt-Wirkung gegen saugende Insekten insbesondere gegen saugende Insekten der Ordnung Homoptera und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Diese Wirkstoffe zeignen auch eine sehr günstige Wirkung gegen Fliegen, wie z.B. Musca Domestica und Mückenlarven. Ferner zeichnen sie sich durch eine breite ovizide und ovilarvizide Wirkung aus und besitzen eine wertvolle Wirkung gegen ektoparasitäre Milben und Zecken z.B. der Familien Ixodidae, Argasidae und Dermanyssidae.

Die Verbindungen der Formel I sowie das 2-[Pyridyl-(3')]-thiazolin und dessen Salze werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen,

Spritzpulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder

organischer Natur wie insbesondere Dolomit oder zerkleinerte Pflanzenrückstände verwendet werden.

Als oberflächenaktive Verbindungen kommen je nach der Art des zu formulierenden Wirkstoffes nichtiogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die

Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im(aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95% Wirkstoff, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe

(% = Gewichtsprozent)

| 1. Emulsions-Konzentrate | a) | b) | c) |
|---|---|---|---|
| Wirkstoff | 25% | 40% | 50% |
| Ca-Dodecylbenzolsulfonat | 5% | 8% | 6% |
| Ricinusöl-polyäthylenglykoläther (36 Mol AeO) | 5% | - | - |

| | | | |
|---|---|---|---|
| Tributylphenol-polyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe

(% = Gewichtsprozent)

| 5. Spritzpulver | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykoläther ( 15 Mol AeO) | 6% |
| Na-Lingninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung von 2-[Pyridyl-(3')]-thiazolin

Eine Lösung von 10,4 g Nicotinsäurenitril und 8 g Cysteamin in 40 ml absolutem Alkohol wird während 3 Stunden zum Sieden unter Rückfluss erhitzt. Anschliessend dampft man die Lösung im Vakuum bei 50°C Badtemperatur ein und destilliert den Rückstand im Hochvakuum.

Man erhält die Verbindung der Formel

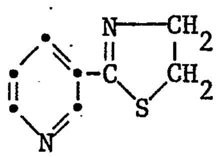 (1)

mit einem Siedepunkt von 90°C bei 0,1 mm/Hg.

Auf analoge Weise werden auch folgende Verbindungen hergestellt:

(2) 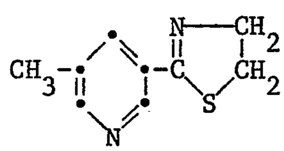 Smp. 53-55°C

(3) 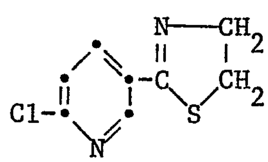 Smp. 77-78°C

(4) 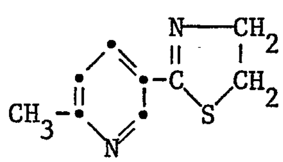 Smp. 52-54°C

(5) 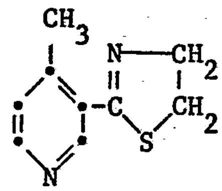 Sdp. 95-98°C/0,05Pa

(6) 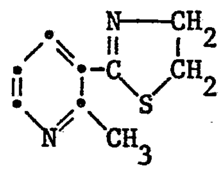 $n_D^{20°} = 1{,}5930$

(7) 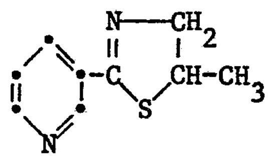 Sdp. 68°C/0,07Pa

(8) 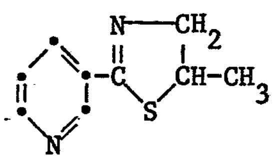 • HCl Smp. 148-150°C

(9) 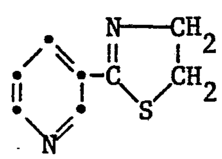 • HCl Smp. 193°C

(10) 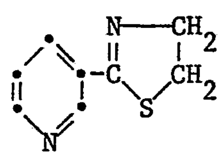 • $H_2SO_4$ • $2H_2O$ Smp. 150-153°C

<u>Beispiel 2:</u> <u>Insektizide systemische Wirkung: Aphis craccivora</u>

Bewurzelte Bohnenpflanzen werden in Töpfe, welche 600 ccm Erde enthalten, verpflanzt. Anschliessend werden 50 ml einer Versuchslösung mit 25 ppm, 5 ppm bzw. 1 ppm der zu prüfenden Verbindung direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei Pflanzen, je eine in einem separaten Topf, verwendet. Der Versuch wird bei 25°C und 70% relativer Luftfeuchtigkeit durchgeführt.

Die Verbindungen gemäss Beispiel 1 haben die in der folgenden Tabelle angegebene Wirkung gegen Aphis craccivora.

Biologische Versuchsergebnisse

In der folgenden Tabelle sind Versuchsergebnisse auf der Basis des vorstehenden Beispiels aufgeführt, und zwar mit folgendem Bewertungsindex in Bezug auf die prozentuale Abtötung der Schädlinge:

A: 70-100% Abtötung bei 1 ppm Wirkstoffkonzentration
B: 70-100% Abtötung bei 5 ppm Wirkstoffkonzentration
C: 70-100% Abtötung bei 25 ppm Wirkstoffkonzentration

| Verbindung Nr. | Wirksamkeit gegen Aphis craccivora |
|---|---|
| 1 | A |
| 2 | B |
| 3 | B |
| 4 | B |
| 5 | C |
| 6 | C |
| 7 | A |
| 8 | C |
| 9 | C |
| 10 | C |

Patentansprüche

1. Ein 2-[Pyridyl-(3')]-thiazolin der Formel

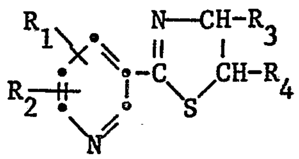

• $(A)_n$ (I) ,

worin $R_1$ und $R_2$ je Wasserstoff, Halogen oder $C_1$-$C_4$-Alkyl,
$R_3$ und $R_4$ je Wasserstoff oder $C_1$-$C_4$-Alkyl,
A eine anorganische oder organische Säure und
n die Zahlen 0, 1 oder 2 bedeuten, mit der Massgabe, dass
$R_1$, $R_2$, $R_3$ und $R_4$ nicht zusammen Wasserstoff sein dürfen.

2. Eine Verbindung gemäss Anspruch 1, worin $R_1$ Wasserstoff, $R_2$ Chlor oder Methyl und $R_3$ und $R_4$ je Wasserstoff oder $R_1$ und $R_2$ je Wasserstoff, $R_3$ Wasserstoff und $R_4$ Methyl oder Aethyl oder $R_3$ Methyl oder Aethyl, $R_4$ Wasserstoff, A eine anorganische oder organische Säure und n die Zahlen 0, 1 oder 2 bedeuten.

3. Die Verbindung gemäss Anspruch 2 der Formel

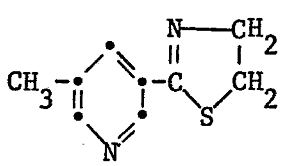

.

4. Die Verbindung gemäss Anspruch 2 der Formel

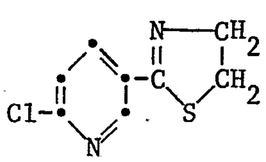

.

5. Die Verbindung gemäss Anspruch 2 der Formel

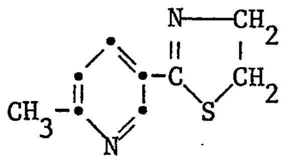 .

6. Die Verbindung gemäss Anspruch 2 der Formel

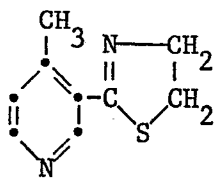 .

7. Die Verbindung gemäss Anspruch 2 der Formel

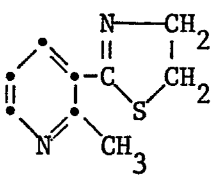 .

8. Die Verbindung gemäss Anspruch 2 der Formel

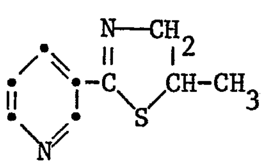 .

9. Die Verbindung gemäss Anspruch 2 der Formel

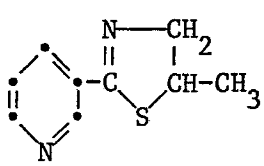 • HCl .

10. Verfahren zur Herstellung von Verbindungen gemäss Anspruch 1, dadurch gekennzeichnet, dass man

a) eine Verbindung der Formel

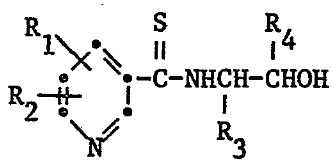

mit Thionylchlorid ringschliesst oder

b) eine Verbindung der Formel

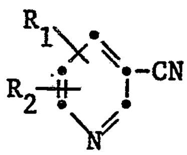

mit einer Verbindung der Formel

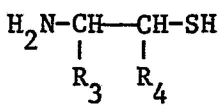

reagieren lässt, worin $R_1$ bis $R_4$ die im Anspruch 1 angegebene Bedeutung haben.

11. Ein Schädlingsbekämpfungsmittel, welches neben geeigneten Träger und/oder Zuschlagstoffen als aktive Komponente eine Verbindung gemäss Anspruch 1 sowie 2-[Pyridyl-(3')]-thiazolin oder dessen Salze mit anorganischen oder organischen Säuren enthält.

12. Die Verwendung von Verbindungen gemäss Anspruch 1 sowie von 2-[Pyridyl-(3')]-thiazolin oder dessen Salzen mit anorganischen oder organischen Säuren zur Bekämpfung von Schädlingen an Tieren und Pflanzen.

13. Die Verwendung gemäss Anspruch 12 zur Bekämpfung von Insekten.

0062612

Europäisches Patentamt

# EUROPÄISCHER RECHERCHENBERICHT

Nummer der Anmeldung

EP 82 81 0141

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-4 080 457 (HARRISON et al.)<br>* Spalten 1-5 *<br>--- | 1,11-13 | C 07 D 417/04<br>A 01 N 43/78 |
| Y | FR-A-2 356 423 (OERIU)<br>* Seiten 1,2 *<br>--- | 1 | |
| P | US-A-4 260 765 (HARRISON et al.)<br>* Spalten 1-3 *<br>--- | 1,11-13 | |
| A | CH-A- 433 322 (VEB ARZNEIMITTELWERK DRESDEN)<br>--- | | |
| A | EP-A-0 010 420 (ELI LILLY AND CO:)<br>----- | | RECHERCHIERTE SACHGEBIETE (Int. Cl. 3)<br>C 07 D 417/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 14-06-1982 | BRIGHENTI L.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82